Europäisches Patentamt

European Patent Office

Office européen des brevets

(11) Publication number: **0 340 005**
A2

(12) **EUROPEAN PATENT APPLICATION**

(21) Application number: **89304220.0**

(22) Date of filing: **27.04.89**

(51) Int. Cl.⁴: **A 61 K 37/02**

(30) Priority: **27.04.88 US 186538**

(43) Date of publication of application:
**02.11.89 Bulletin 89/44**

(84) Designated Contracting States:
**AT BE CH DE ES FR GB GR IT LI LU NL SE**

(71) Applicant: **IMMUNOMEDICS, INC.**
**150 Mt. Bethel Road, CN 4918**
**Warren New Jersey 07060 (US)**

(72) Inventor: **Goldenberg, Milton David**
**397 Long Hill Drive**
**Short Hills New Jersey 07078 (US)**

(74) Representative: **Sheard, Andrew Gregory et al**
**Kilburn & Strode 30, John Street**
**London WC1N 2DD (GB)**

The title of the invention has been amended (Guidelines for Examination in the EPO, A-III, 7.3).

(54) **Use of cytokines for improvement of radiotherapy.**

(57) External beam radiation therapy of cancer and other abnormal proliferative processes, and immunosuppression of transplant candidates, is improved using cytokines to prevent, mitigate or reverse adverse radiation-induced toxicity, especially to hematopoietic cells. A preferred cytokine is IL-1. Higher doses of radiation can be administered and tolerated by the patient and dose-limiting marrow toxicity can be prevented, palliated or reversed using adjunct cytokine therapy.

EP 0 340 005 A2

Description

# IMPROVED RADIOTHERAPY

## BACKGROUND OF THE INVENTION

The present invention relates to an improved method of therapy of cancer and other abnormal proliferative processes, and immunosupppressive treatment of transplant candidates, with external beam radiation, wherein cytokines are used to prevent, mediate or reverse radiation-induced toxicity. especially to hematopoietic cells.

Nonsurgical therapy of cancer and other abnormal proliferative processes using external beam irradiation is limited in its efficacy because of toxic side effects to normal tissues and cells, as a consequence of the limited specificity of this treatment modality for cancer or other abnormally proliferating cells. In acute radiation injury, there is destruction of lymphoid and hematopoietic compartments as a major factor in the development of septicemia and subsequent death.

Many different approaches have been undertaken to protect an organism from the side effects of radiation. One approach is to replace bone marrow cells after toxicity has developed. Another is to give agents which affect DNA repair mechanisms such as the chemical radioprotection afforded by thiol compounds.

Neta et al. (J. Immunol. 136:2483-2485, 1986) showed that pre- treatment with recombinant interleukin-1 (IL-1) protects mice in a dose-dependent manner from the lethal effects of external beam irradiation, when the IL-1 was given 20 hr before irradiation. Administering IL-1 4 hr before irradiation significantly reduced the radioprotective effects of IL-1. However, IL-1 cannot be administered too long before irradiation, because these authors also found that at 45 hr before irradiation, a drastic reduction in survival. as compared to the mice given IL-1 at 20 hr before irradiation, was achieved. Thus, this study indicated that IL-1 should be given at a critical period before lethal irradiation.

This was the first evidence that a cytokine, which acts as a differentiation-inducing and maturation-inducing agent for a variety of cells, can initiate radioprotective events in vivo when given prior to external beam irradiation. However, other kinds of immunomodulators have been reported to confer radioprotection. Numerous impure microbial components. such as lipopolysaccharide, which are now recognized to enhance hematopoietic and immune functions, were shown to have radio-protective activity more than thirty years ago (Smith et al., Am J. Physiol. 109:124-130, 1957; Mefford et al., Proc. Soc. Exp. Biol. Med. 83:54-63, 1953; Ainsworth and Chase, Proc. Soc. Exp. Biol. Med. 102:483-489, 1959).

The effects of IL-1 are mediated through the induction of colony stimulating factor (CSF) (Vogel et al., J. Immunol. 138:2143-2148, 1987), one of many hematopoietic growth factors induced by IL-1 stimulation of endothelial cells (Broudy et al., J. Immunol. 139:464-468, 1987; Lee et al., Exp. Hematol. 15:983-988, 1987; Takacs et al., J. Immunol. 138:2124-2131, 1985). However, it was shown by Neta et al. (Lymphokine Res. 5:s105, 1986; J. Immunol. 140:108-111, 1988) that human recombinant granulocyte CSF (rG-CSF) or granulocyte-macrophage CSF (GM-CSF) alone do not confer radioprotection, but do work synergistically with IL-1 to prevent radiation death in mice. Interestingly, this study also showed that mouse strains react differently to radiation and the radioprotection of IL-1, thus making extrapolation of such effects to other species, especially humans, difficult. This lack of radiation protection by the CSF's alone is in contrast to their being able to induce a recovery of neutropenia in mice treated with the anticancer drug, 5-fluorouracil (5-FU) (Moore and Warren, Proc. Natl. Acad. SCi. USA 84:7134-7138, 1987). Likewise, these authors reported that the neutropenic effects of 5-FU could be reduced by treating the mice with the cytokine 4 hr after giving the 5-FU, and that there was a synergy of IL-1 and G-CSF in acceleration of neutrophil regeneration. These studies indicate, when compared to the work of Neta et al. (cited above) for radiation protection, that different time schedules are needed for IL-1 application in drug-induced or external beam irradiation-induced myelosuppression, and that the cytokines can act differently in their ability to prevent radiation- or chemotherapy-induced myelosuppression.

Although it has been shown that an important function of IL-1 is as an immune stimulator, a plethora of other properties have been ascribed to this substance, as contained in the reviews of Dinarello (Rev. Infectious Dis. 6:51-95, 1984; Oppenheim et al., Immunology Today 7:45-56, 1986; Lomedico et al., Cold Spring Harbor Symp. Quantit. Biol. 51:631-639, 1986):

1. Stimulation of mouse thymocyte activation (Lomedico et al., Nature 312:458, 1984);

2. Stimulation of human dermal fibroblast proliferation (Dukovich et al., Clin. Immunol. Immunopathol. 38:381, 1986; Gubler et al., J. Immunol., 136:2492, 1986);

3. Stimulation of IL-2 production (Kilian et al., J. Immunol. 136:4509, 1986);

4. Stimulation of $PGE_2$ and collagenase production by human rheumatoid synovial cells and dermal fibroblasts (Dukovich et al., Clin. Immunol. Immunopathol. 38:381, 1986; Gubler et al., J. Immunol. 136:2492, 1986);

5. Stimulation of arachidonic acid metabolism in liver and smooth muscle cells (Levine and Xiao, J. Immunol. 135:3430, 1985);

6. Stimulation of metallothionein gene expression in human hepatoma cells (Karin et al., Mol. Cell. Biol. 5:2866, 1985);

7. Stimulation of synthesis of certain hepatic acute-phase proteins (Bauer et al., FEBS Lett. 190:271, 1985; Ramadori et al., J. Exp.

Med. 162:930, 1985; Perlmutter et al., Science 232:850, 1986; Westmacott et al., Lymphokine Res. 5:87, 1986);

8. Stimulation of bone resorption in vitro (Gowen and Mundy, J. Immunol. 136:2478, 1986);

9. Stimulation of ACTH production in a pituitary tumor cell line (Woloski et al., Science 230: 1035, 1985);

10. Cachectin-like activity (tumor necrosis factor) to suppress lipoprotein lipase activity in adipocytes (Beutler et al., J. Exp. Med. 161:984, 1985);

11. Activity as B-cell growth and differentiation factor (Pike and Nossal, Proc. Natl. Acad. Sci. USA 82:8153, 1985);

12. Stimulation of platelet-activating factor production in cultured endothelial cells (Bussolino et al., J. Clin Invest. 77:2027, 1986); and

13. Stimulation of monocyte- or T-cell-mediated tumor cell cytotoxicity (Lovett et al, J. Immunol.136:340-347, 1986; Onozaki et al., J. Immunol. 135: 314-320, 1985; Farrar et al., J. Immunol. 123:1371-1377, 1980).

Whereas the above listing refers to in vitro effects of IL-1, IL-1 in vivo has been shown in rodents to (1) be pyrogenic (Mccarthy et al., Am. J. Clin. Nutr. 42:1179, 1985; Tocco-Bradley et al., Proc. Soc. Exp. Biol. Med. 182:263, 1986), (2) promote leukocytosis and hypozincemia (Tocco-Bradley et al., Proc. Soc. Exp. Biol. Med. 182:263, 1986), and hypoferremia (Westmacott et al., Lymphokine Res. 5:87, 1986), (3) induce a transient suppression of food intake (McCarthy et al., Am. J. Clin. Nutr. 42:1179, 1985), (4) stimulate accumulation of procoagulant activity in endothelial cells (Nawroth et al. Proc. Natl. Acad. Sci. USA 83:3460, 1986), (5) induce a local inflammatory response in the skin (Granstein et al., J. Clin. Invest. 77:1020, 1986), and (6) inhibit the growth of murine syngeneic tumors (Nakamura et al., Gann 77:1734-1739, 1986). Therefore, the demonstration that mice can be protected from lethal doses of external beam irradiation by prior administration of IL-1 (Neta et al., J. Immunol. 136:2483, 1986), alongside all these other actions, does not predict that it will be useful in humans when given prior to, during and/or after external beam irradiation.

Cancer therapy using external beam radiotherapy is well known. Two basic types of radiation therapy are currently available; brachytherapy and teletherapy. The former involves the placement of the radiation device within or near the target tissue (e.g., interstitial and intracavitary radiation used in the treatment of many gynecologic and oral cancers), whereas teletherapy uses a device which is more removed from the patient, as in most orthovoltage or supervoltage machines. With the development of betatrons and high-energy accelerators, electron beam therapy has become available for teletherapy.

External beam radiation is also used for immunosuppression of transplant candidates. Other non-cancer uses of radiation therapy include ectopic bone formation (e.g., in the hip) after trauma, cheloids gyneco-mastia in males receiving estrogen therapy multiple scelrosis, spleen in idiopathic thrombocytopenic purpura, ectropion, and the like, where radiation is intended to control an abnormal proliferative process.

A need therefore continues to exist for methods of preventing, mitigating or reversing toxicity to myeloid and hematopoietic cells, which is a limiting side effect of treatment of cancer and allied diseases in humans with external beam radiotherapy.

## OBJECTS OF THE INVENTION

One object of the present invention is to provide an improved method of radiotherapy in cancer and other abnormal proliferative processes, and for immunosuppression of transplant candidates, wherein hematopoietic or myeloid toxicity produced as a side effect of exposure to external beam radiation therapy can be prevented, mitigated or reversed.

Another object of the present invention is to permit higher doses of radiation to be administered to and tolerated by patients without unacceptable damage to myeloid and other hematopoietic cells and resultant dangerously low white blood cell (WBC) levels.

Upon further study of the specification and appended claims, further objects and advantages of this invention will become apparent to those skilled in the art.

## SUMMARY OF THE INVENTION

These objects are achieved, in a method of external beam radiotherapy of cancer, or another abnormal proliferative process, or for immunosuppression of a transplant candidate, wherein a human patient suffering from an abnormal proliferative lesion, or a transplant candidate, susceptible to treatment with external beam radiation therapy, is treated with a therapeutic dose of external beam radiation which also produces hematopoietic or myeloid toxicity, by the improvement which comprises administering to the patient an amount effective for substantially preventing, mitigating or reversing such hematopoietic or myeloid toxicity of a differentiation/maturation-inducing cytokine, prior to, simultaneously with, or subsequent to exposure to the radiation.

## DETAILED DISCUSSION

The radioprotective effect of IL-1 in certain murine strains was shown by Neta et al. for external beam irradiation by cobalt-60 gamma rays. These authors observed radioprotective activity of IL-1 in lethally irradiated mice when the cytokine was given 20 hours before irradiation, whereas giving it earlier or later did not result in a similar protection. In light of the varied physiological effects of cytokines and the limited clinical data for their use in humans, it could not be predicted whether particular cytokines might have any radioprotective effects in humans or, if so, when and how they should be administered to achieve such effects. In particular, use of cytokines to mitigate or reverse hemato- poietic toxicity when administered during or after radiation therapy could not be predicted in humans, nor could the efficacy of other cytokines than IL-1.

Moreover, the aforementioned work of Moore and Warren indicates that different time schedules are needed for IL-1 to have its protective effects in radiation-induced myelosuppression. Further, other growth factors, such as G-CSF and GM-CSF, behave differently in radiation or drug-protection, suggesting that the myelosuppression may be different. This previous work could not predict that prior, simultaneous or subsequent administration of a cytokine in humans would mitigate or reverse myeloid or hematopoietic toxicity.

Cytokines, or growth factors, are hormone-like peptides produced by diverse cells and are capable of modulating the proliferation, maturation and functional activation of particular cell types. Herein, cytokines refer to a diverse array of growth factors, such as hematopoietic cell growth factors (e.g., erythropoietin, colony stimulating factors and interleukins), nervous system growth factors (e.g., glial growth factor and nerve growth factor), mostly mesenchymal growth factors (e.g., epi- dermal growth factor), platelet-derived growth factor, and fibroblast growth factor I, II and III, but in this invention excluding interferons.

It will be appreciated that there may be several cytokines that are involved in inducing cell differentiation and maturation, and that cytokines may have other biological functions. This also makes it difficult to predict whether a safe and restricted biological effect can be achieved in humans, such as prevention, mitigation or reversal of myelosuppres- sion. In the case of IL-1, there may be several forms, such as IL-1-alpha and IL-1-beta, which nevertheless appear to have a similar spectrum of biological activity. Preferred cytokines for use in the method and compositions of the invention are lymphokines, i.e., those cytokines which are primarily associated with induction of cell differentiation and maturation of myeloid and possibly other hematopoietic cells. A preferred lymphokine is IL-1. Other such lymphokines include, but are not limited to, G-CSF, M-CSF, GM-CSF, Multi-CSF (IL-3), and IL-2 (T-cell growth factor, TCGF). IL-1 appears to have its effect mostly on myeloid cells, IL-2 affects mostly T-cells, IL-3 affects mutiple precursor lymphocytes, G-CSF affects mostly granulocytes and myeloid cells, M-CSF affects mostly macrophage cells, GM-CSF affects both granulocytes and macrophage. Other growth factors affect immature platelet (thrombocyte) cells, erythroid cells, and the like.

The cytokines can be used alone or in combination to protect against, mitigate and/or reverse myeloid or hematopoietic toxicity associated with radiation. Examples of possible combinations include IL-1 + GC-CSF, IL-1 + IL-3, G-CSF + IL-3, IL-1 + platelet growth factor and the like. Certain combinations will be preferred, depending on the maturation state of the target cells to be affected, and the time in the course of radiotherapy that the protective agent needs to be administered. For example, in patients with depression of several hematopoietic cell types (e.g., myeloid, lymphoid and platelet), a combination of IL-1 + IL-3/and/ or platelet growth factor is preferred, while more severe depression of the myeloid series may require such combinations as IL-1 + G-CSF. Radia- tion can have greater compromising effects on particular hematopoietic elements, either because of the type of lesion or the dosage necessary to achieve a therapeutic effect, and the appropriate choice, dosage and mode of administration of cytokine(s) will follow from such effects.

As mentioned, depending upon the circumstances, an appropriate dose of the cytokine can be administered prior to, simultaneously with or subsequent to the administration of the radiation therapy. The object will be to maximize the cytotoxic activity of the radiation on the lesion, while minimizing toxicity to the myeloid and other hematopoietic cells. Careful monitoring of the WBC and other blood elements, including but not limited to erythrocyte (red blood cell/RBC) count, thrombocyte (platelet) count, and including a differential WBC analysis to monitor the myloid/ lymphoid series, as well as the bone marrow hematological picture during the course of therapy, with particular attention to possible depletion of myeloid lymphoid forms, but also the status of immature erythrocytes, myelocytes, lymphocytes and thrombocytes, will permit optimization of the cytokine treatment. Depending upon which hematologic element is adversely affected, the choice of cytokine and administration schedule can be individualized in each circumstance, including the combination of cytokines, such as IL-1 + IL-3, Il-1 + IL-2, IL-1 + GM-CSF, IL-1 + platelet growth factor and the like.

Correlation of the choice of cytokine, singly or in combinations, and doses thereof, to hematotoxicity is important, since each cytokine generally has its effect mostly on particular hematopoietic elements. For example, if a cytotoxic agent has both severe myeloid and thrombocytic toxicity, the combination of IL-1 and IL-3 in a 1:1 or 2:1 (or higher) ratio will be advantageous. Thus, reduction in the WBC count to a level below about 2,000 and platelets to a level below about 20,000 can be reversed by administration of from about 1 ug to about 500 ug, preferably 5-100 ug, more preferably about 10ug of rIL-1 in a single dose, together with or followed by administration of from about 1 ug to about 200 ug, preferably 5-50 ug, more preferaby about 5 ug of IL-3. The applications can be repeated, with the reversal of the myeloid and platelet depressions occurring within about 5-20 days, usually about 7 days. The ordinary skilled clinician will appreciate that variations in the timing and dosage of cytokine administration and cytokine combinations and dosages are a function of the cytokine used, the nature of the bone marrow and/or other hematopoietic element depressed, and the nature of the patient (e.g., prior toxicity affecting bone marrow status) and the cytotoxic agent and protocol.

The method of this invention is particularly useful for the treatment of cancers of the head and neck, lung, esophagus, pancreas, breast and of the hepatobiliary system, as well as neoplasms of the mediastinum, colorectal cancer, cancers of the urogenital system, sarcomas of the bone, lymphomas (including Hodgkin's Disease), ectopic bone formation after trauma, cheloids, gynecomastia in males receiving estrogen therapy, multiple sclerosis, spleen in idiopathic thrombocytopenic purpura, ectropion and similar non-cancerous abnormal proliferative processes. In addition, external beam radiation is used for immunosuppression of candidates for organ or tissue transplants, including marrow transplants, and the use of cytokine adjuvant therapy according to the method of this invention reduces the hematopoietic toxicity and myelotoxicity of such treatment.

It is well known in the art that various methods of radiation therapy can be used for the treatment of cancer and other pathological proliferative processes. A clinician experienced in these procedures will readily be able to adapt the cytokine adjuvant therapy described herein to such procedures, to mitigate marrow suppression and other such hepatopoietic side effects by administration of the cytokine before, during and/or after radiation therapy.

The mode of administration of the radiation therapy as well as of the cytokine should be coordinated and optimized. It is important to consider the concept of therapeutic index in delivering the radiation therapy, that is, the relationship between desired and undesired effects. The skilled radiotherapist uses a number of well-known methods of varying the timing and dosage of radiation to take advantage of increasing target kill while protecting other, non-target sites. These include, e.g., fractionation, protraction, split course technique, interstitial treatment, and manipulation of the target volume (see, e.g., Hellman, S., pp. 227-255, in Devita et al., CANCER, Principles and Practice of Oncology, 2nd ed., 1985, J.B. Lippincott Co., Philadelphia).

In general, most radiotherapists administer conventional radiation fractions of 180 to 250 rad per day. This is consistent with tumor control without excessive acute or late effects. However, under cytokine control, larger fractionated doses can be tolerated in the treatment of a number of target lesions. Also, when giving large doses of radiation, e.g., in the range of 2,000-5,000 rad or higher, it is usually necessary to delay the next dose for about 4-6 weeks in order to allow the tissues to recover from radiation injury. However, with the institution of cytokine adjuvant therapy according to the present invention, shorter intervals between high dose radiation are necessary, thus providing advantages for the patient and for the therapy protocol.

It will be appreciated that biological considerations of localized radiation affect its efficacy. High-dose, whole-body radiation has a profound effect on the immune response. However, this generalized treatment is rarely use in clinical radiation therapy, except as preparation for bone marrow transplantation, where the cytokine adjuvant therapy of the invention is especially useful. Localized radiation produces a chronic leukopenia, particularly affecting both T and B lymphocytes.

Normally, marrow suppression will call for cytokine treatment that restores as much of the depressed hematopoietic and myeloid activity as possible, which can be achieved by use of individual cytokines or combinations thereof, as disclosed hereinabove. In the special case where radiotherapy is used to effect immunosuppression of a transplant candidate such as a bone marrow transplant candidate, the purpose of the radiation is to suppress T lymphocyte levels, since these are the cells that mount an immune response resulting in rejection of the foreign cells or tissues. It is desirable to restore granulocyte and platelet activity, as well as other hematopoietic and myeloid activity that protects the patient from infection and other complications, but does not compromise the anti-rejection function of the radiation-induced immunosuppression. This can be achieved with cytokines that stimulate production of granulocytes (e.g., G-CSF, GM-CSF), platelets, B lymphocytes and other hematopoietic and myeloid components without inducing T cell proliferation, and such would be the preferred treatment agents in such circumstances.

Administration of the cytokine is advantageously effected intravenously to have its maximum effect on the circulation through the bone marrow. Other forms of administration of the cytokine, e.g., intraarterial, intrathecal, may be advantageous for more immediate access to the region of greatest hematopoietic cell compromise.

A further parameter to consider is whether the cytokine is administered in a single dose, in multiple doses or as a continuous infusion during the course of therapy. Certain cytokines are cleared rapidly from the body and will require periodic or continuous administration in order for their efficacy to be maximized. Depending if a pre-treatment, intra-treatment, or post-treatment of the cytokine is given, the manner of administration can differ. For example, if the cytokine is given concomitantly with the radiation, it is preferable to administer the cytokine by continuous intravenous infusion over several hours and optionally repeated on one or more days during and after completion of the radiation therapy. It should also be understood that continuous administration of a cytokine can be effected by any of the transdermal modes of drug administration known to the art or yet to be developed.

The dosage level of the cytokine will be a function of the extent of compromise of the hematopoietic cells, correlated generally with the WBC level in the patient. Periodic monitoring of the WBC and other blood cell counts and adjustment of the rate and frequency of infusion or the dosage of the cytokine administered to achieve a relatively constant level of WBC's will ensure that the patient does not sustain undue marrow toxicity from the therapy. Experience will permit anticipation of WBC lowering and infusion of the cytokine at a time and in an amount sufficient to substantially prevent WBC depression. Importantly, this also insures that excessive side effects due to the cytokine itself are not produced, but only such side effects as are necessary to prevent compromise of the patient's myeloid and other hematopoietic cell systems.

Correlation of cytokine dosage to WBC count suggests that, in general, reduction of WBC count from a normal range of 8-12,000/cmm to a level of about 2,000 can be reversed by administration of from about 1 ug to about 500 ug, preferably 5-100 ug, more preferably about 10 ug of recombinant human IL-1 in a single dose, the reversal of WBC count depression occurring within about 2-12 days, usually about 4 days. The clinician will appreciate that variations in the timing and dosage of cytokine administration as a function of the type of cytokine used, the extent and rate of compromise of the bone marrow and/or other components of the myeloid and/or other hematopoietic elements and the individual characteristics of the patient and the therapy protocol will be possible and often desirable. These can easily be made by the clinician using conventional monitoring techniques and dosimetric principles.

The present invention includes administration of one or a combination of cytokines, preferably lymphokines, prior to, together with and/or subsequent to administration of cytotoxic radiation. The guidelines provided herein will enable the ordinary skilled clinician to adapt cytokine administration to enhance the efficacy and mitigate the adverse hematopoietic side effects of cytotoxic therapy as a function of WBC, platelet and erythrocyte counts, marrow component status and other particular diagnostic indicia peculiar to the individual patient. In general, this invention is applicable to support an enhancement of efficacy of any cytotoxic therapy that has serious hematopoietic side effects that limit the therapy's efficacy.

The present invention is practiced using sterile injectable preparations suitable for human use. Where administration of the cytokine is to be intravenous or intraarterial, such solutions are normally prepared in sterile phosphate-buffered saline (PBS), this mode being the preferred one to effect mitigation of bone marrow toxicity.

Kits for therapy according to the present invention will normally include sterile preparations of the appropriate dosage of the cytokine either as a solution or as a solid, advantageously a lyophilized solid, ready for dissolution in the appropriate sterile injection medium, e.g., PBS.

Without further elaboration, it is believed that one skilled in the art can, using the preceding description, utilize the present invention to the fullest extent. The following preferred specific embodiments are, therefore, to be construed as merely illustrative, and not limitative of the remainder of the disclosure in any way whatsoever. In the following examples, all temperatures are set forth uncorrected in degrees Celsius; unless otherwise indicated, all parts and percentages are by weight.

## EXAMPLES

Treatment of Non-Hodgkin's Lymphoma

A 52-year old man presents with a lymphoma in his mediastinum, which histologically is classified as a non-Hodgkin's lymphoma. A radiation dose of 6,000 rad is given to the patient in fractions, 5 days a week, over a period of 3 weeks. At this radiation dose, toxicity usually begins to show by the 15th day from the end of the first week, where a drop in WBC's is noted. By 1-2 weeks after therapy is ended, WBC counts of less than 2,000/cmm are seen. In this circumstance, after the first week of radiotherapy, the patient is infused on two alternate days with two doses of 10 ug IL-1 each. Upon measurement of the WBC count over the next 6 weeks, repetition of the IL-1 therapy is instituted each week as long as the WBC is depressed below 4,000/cmm. As a result of this combination therapy, a shrinkage of the mediastinal lymphoma, as noted by CAT scan, is achieved.

2. Treatment of Bone Cancer

A 24-year old man is diagnosed to have a chondrosarcoma of the right fibula. Since a high radiation dose is desired, the patient is given electron beam therapy to the tumor, combined with GM-CSF therapy. A high radiation dose of 8,000 rad is delivered over 8 weeks, in divided daily doses, 5 times per week. Human recombinant GM-CSF is given at the onset of radiation therapy, by continuous intravenous infusion, at a dose of 12 ug per kg of patient body weight per day. The patient experiences transient myalgia and a rash at this dosage level, but this subsides within 2 weeks post-therapy. The patient's WBC count stays above 4,000/cmm during weeks 6-12 of therapy onset and returns to almost normal levels by 5 weeks after the conclusion of radiation therapy. At this time, a more than 75% reduction in tumor size is noted.

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.

## Claims

1. The use of an amount effective for substantially preventing, mitigating or reversing such hematopoietic or myeloid toxicity of a differentiation/maturation-inducing cytokine, in the preparation of an agent for use in a method of external beam radiotherapy of cancer, or another abnormal proliferative process, or for immunosuppression of a transplant candidate, wherein a human patient suffering from an abnormal proliferative lesion, or a transplant candidate, susceptible to treatment with external beam radiotherapy, is treated with a therapeutic dose of external beam radiation which also produces hematopoietic or myeloid toxicity, wherein said method further comprises administering to said patient an amount effective for substantially preventing, mitigating or reversing such hematopoietic or myeloid toxicity of a differentiation/maturation-inducing cytokine, prior to, simultaneously with, or subsequent to exposure to said radiation.

2. The use according to claim 1, wherein said disease is cancer.

3. The use according to claim 1, wherein said disease is a non-cancerous abnormal proliferative process.

4. The use according to claim 1, wherein said radiotherapy is administered to a transplant candidate.

5. The use according to any of claims 2, 3 or 4, wherein said cytokine is a lymphokine.

6. The use according to any of claims 2, 3 or 4, wherein said cytokine is interleukin-1 (IL-1).

7. The use according to any of claims 2, 3 or 4, wherein said cytokine is a colony stimulating factor (CSF), interleukin-2 (IL-2) or interleukin-3 (IL-3).

8. The use according to any of claims 2, 3 or 4, wherein said cytokine is administered prior to administration of said radiotherapy.

9. The use according to any of claims 2, 3 or 4, wherein said cytokine is administered concomitantly with administration of said radiotherapy.

10. The use according to any of claims 2, 3 or 4, wherein said cytokine is administered subsequent to administration of said radiotherapy.

11. The use according to claim 10, wherein said cytokine is administered at least 3 days after administration of said radiotherapy.

12. The use according to any of claims 2, 3 or 4, wherein a combination of cytokines is administered.

13. The use of a differentiation/maturation inducing cytokine in the preparation of an agent for use in a method of external beam radiotherapy of cancer or other abnormal proliferative process, or immunosuppression of a transplant candidate, in humans.